# EUROPEAN PATENT APPLICATION

(11) **EP 2 719 458 A1**
(43) Date of publication of application: **16.04.2014**
(21) Application number: 13004828.3
(22) Date of filing: 08.10.2013
(51) Int. Cl.: B01L 3/00

(54) **Aliquot Container**

(30) Priority: 09.10.2012 JP 2012224419
(71) Applicant: Aoi Seiki Co., Ltd., Kumamoto-ken (JP)
(72) Inventor: Itoh, Teruaki, Kumamoto-shi, Kumamoto-ken (JP)
(74) Representative: Plöger, Jan Manfred

(57) **Abstract**

An aliquot container (30) includes an opening (30) and a main body section (31). The main body section (31) communicates with an outside through the opening (31) and includes a peripheral portion (34) defining an inner space (34), The inner space (35) is tapered in a direction opposite to the opening (31).

## Description

The present invention relates to an aliquot container for use in dividing a sample into aliquots, for example,

To inspect a liquid sample such as urine, conventionally, the liquid sample is collected in a cup. The liquid sample is aliquoted for inspection as preprocessing, or it is extracted from the cup by an amount necessary for inspection, This method is disclosed in Jpn. Pat. Appln. KOKAI Publication No. 2005-233765.

If the amount of liquid collected as a sample in a cup is small, the liquid spreads out on the undersurface of the cup. This sample is likely to be difficult to aliquot with a nozzle and, in this case, it is aliquoted manually using a dropper.

According to an aspect of embodiments, an aliquot container comprises an opening and a main body section. The main body section communicates with an outside through the opening and includes a peripheral portion defining an inner space. The inner space is tapered in a direction opposite to the opening.

The accompanying drawings, which are incorporated in and constitute a part of the specification, illustrate embodiments of the invention, and together with the general description given above and the detailed description of the embodiments given below, serve to explain the principles of the invention.
FIG. 1 is a perspective view showing part of a sample preprocessing apparatus including an aliquot container according to a first embodiment of the present invention;
FIG. 2 is a cross-sectional view of the sample preprocessing apparatus, taken along line F2-F2 of FIG. 1;
FIG. 3 is a plan view of the aliquot container;
FIG. 4 is a side view of the aliquot container; and
FIG. 5 is a cross-sectional view of a sample preprocessing apparatus including an aliquot container according to a second embodiment of the present invention, which is incorporated in a collection cup, as shown in FIG. 2.

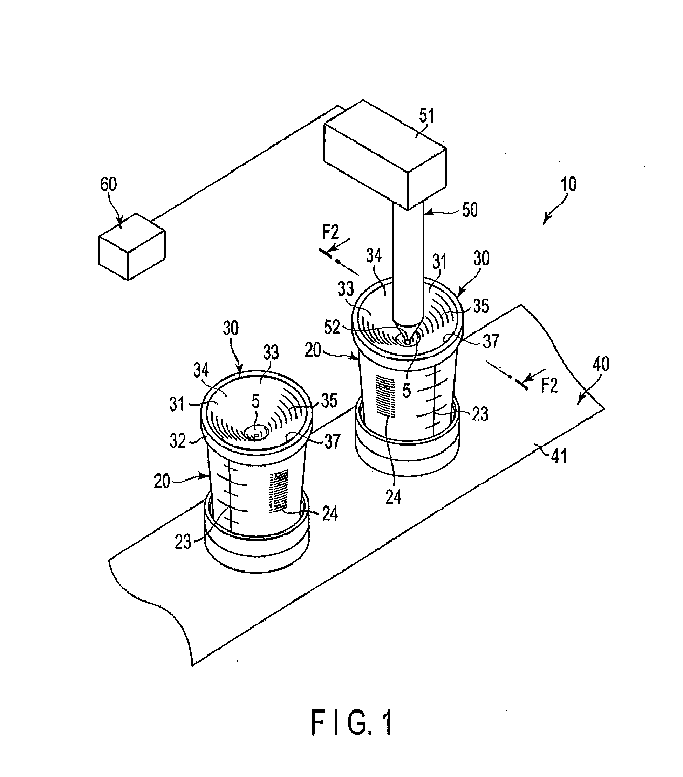

A sample preprocessing apparatus including an aliquot container according to a first embodiment of the present invention will be described with reference to FIGS. 1-4. FIG. 1 is a perspective view showing part of a sample preprocessing apparatus 10. The sample preprocessing apparatus 10 has a function of aliquoting a liquid sample 5 and dispensing it into an inspection container. The aliquoting is dividing a sample into portions and the dispensing is transferring an aliquoted sample portion into another container. In the first embodiment, urine is taken as an example of the liquid sample.

In a place different to the sample preprocessing apparatus 10, the sample 5 is collected in a collection cup 20 and then transferred into an aliquot container 30 therefrom. The aliquot container 30 will be described in detail later.

The collection cup 20 is shaped like a cylinder and has an opening 21 at one end and an undersurface 22 at the other end. The shapes of the opening 21 and undersurface 22 are substantially the same and so are the areas thereof, The collection cup 20 also has on its lateral surface a scale 23 for recognizing the amount of sample and a barcode 24 for representing information about the sample.

The aliquot container 30 into which the sample 5 is transferred and the collection cup 20 in which the sample 5 was originally collected are formed integrally as one unit. Accordingly, the information represented by the barcode 24 provided on the lateral surface of the collection cup 20 coincides with the information of the sample 5 in the aliquot container 30 incorporated into the collection cup 20.

As shown in FIG. 1, the sample preprocessing apparatus 10 comprises a conveyance unit 40 for conveying the collection cup 20 and the aliquot container 30 which are formed integrally as one unit, as described above, an aliquot/dispense unit 50 for aliquoting the sample 5 and dispensing an aliquoted sample portion into an inspection container, and a control unit 60 for controlling the conveyance unit 40 and the aliquot/dispense unit 50,

The conveyance unit 40 includes a conveyor belt 41. The conveyor belt 41 is moved by a driving mechanism or the like. The collection cup 20 and aliquot container 30 are fixed on the conveyance belt 41. As the conveyor belt 41 is moved, the collection cup 20 and aliquot container 30 are conveyed together.

The aliquot/dispense unit 50 includes a nozzle 52. The nozzle 52 is configured to move as a moving arm 51 operates under the control of the control unit 60.

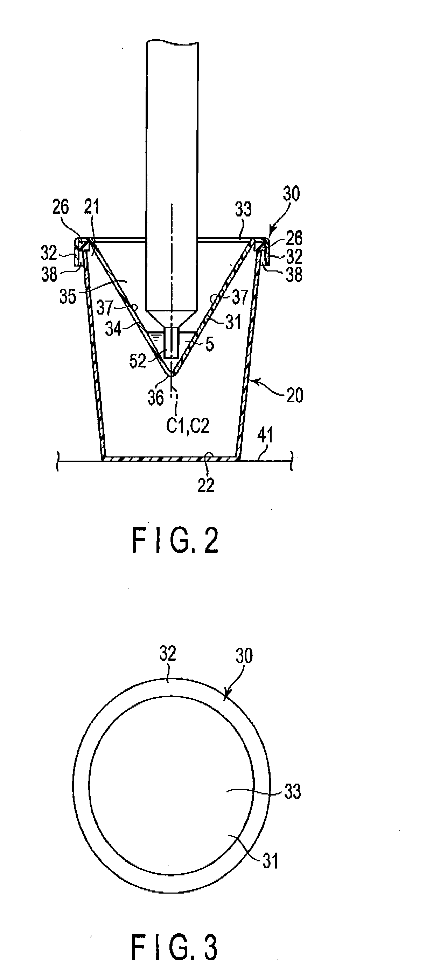

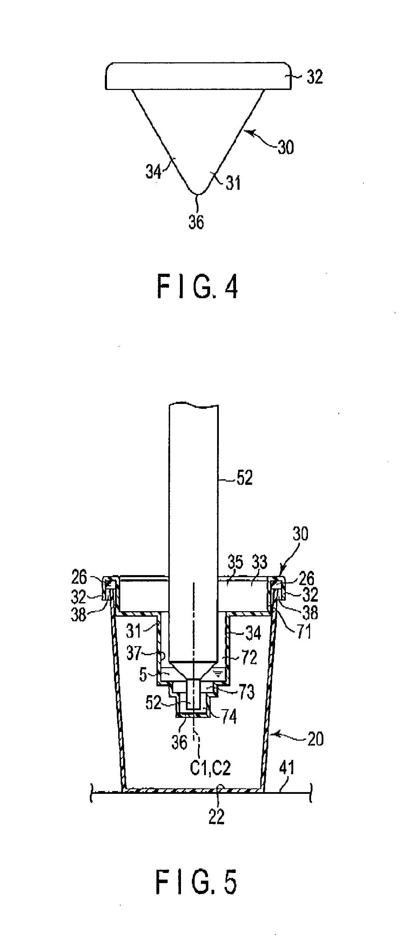

The aliquot container 30 will be described specifically. FIG. 2 is a cross-sectional view of the sample preprocessing apparatus 10, taken along line F2-F2 of FIG. 1. FIG. 2 is a cross-sectional view which shows a situation that the aliquot container 30 is incorporated into the collection cup 20. In FIG. 2, the nozzle 52 is not shown by means of a cross section. FIG. 3 is a plan view of the aliquot container 30 and FIG. 4 is a side view thereof.

As shown in FIGS. 2-4, the aliquot container 30 includes a main body section 31 and a fitting peripheral edge portion 32. The main body section 31 has an opening 33 through which the inside and outside of the main body section 31 communicate with each other. The main body section 31 is shaped like a cone and tapered toward its point 36 from the opening 33.

The main body section 31 includes a cone-shaped peripheral portion 34 on its inner side, as shown in FIG. 2. The main body section 31 includes a housing space 35 surrounded by the peripheral portion 34, The cross section of the housing space 35, which is taken along the line perpendicular to a center line C1, is a circle. The center line C1 is a line passing through the center of the cross section of the housing space 35. This cross section decreases in area toward the point 36 from the opening 33, or the circle gradually decreases in area.

The peripheral portion 34 has an inner surface 37 that extends linearly toward the point 36 of the main body section 31. This linear extension means that even though the aliquot container 30 is cut anywhere to have a planar surface which passes through the center line C1 and is parallel with the center line C1, the inner surface 37 extends linearly toward the point 36 as shown in FIG. 2.

The fitting peripheral edge portion 32 is formed on the outer edge of the opening 33 and bent toward the point 36 from the outer edge of the opening 33. Accordingly, a gap 38 is formed between the main body section 31 and the fitting peripheral edge portion 32. The gap 38 is shaped like a ring around the center line C1.

As shown in FIG. 2, the aliquot container 30 is incorporated into the collection cup 20 such that a peripheral edge portion 26 of the collection cup 20 is included in the gap 38 between the main body section 31 and the fitting peripheral edge portion 32.

It is favorable that the aliquot container 30 should be formed on the basis of the size of the collection cup 20. More specifically, as seen from FIG. 2, it is favorable that the size of the opening 33 of the aliquot container 30 and that of the opening 21 of the collection cup 20 should be substantially the same. It is also favorable that the aliquot container 30 should be formed such that the peripheral edge portion 26 of the collection cup 20 is fitted into the gap 38 between the main body section 31 and the fitting peripheral edge portion 32.

When the aliquot container 30 so configured is incorporated into the collection cup 20, the main body section 31 enters into the collection cup 20 and the center line C1 of the aliquot container 30 coincides or substantially coincides with a center line C2 of the collection cup 20. Thus, the attitude of the the collection cup 20 and aliquot container 30, which are integrally formed as one unit, is stabilized.

An operation of the sample preprocessing apparatus 10 according to the first embodiment will be described. First, the liquid sample 5 collected in the collection cup 20 is transferred into the aliquot container 30. Then, the aliquot container 30 is incorporated into the collection cup 20. Thus, the barcode 24 provided on the lateral surface of the collection cup 20 represents information of the sample 5 transferred into the aliquot container 30.

The operation of transferring the sample 5 can be performed by hand or by the sample preprocessing apparatus 10. To perform the operation, the sample preprocessing apparatus 10 includes a mechanism of transferring the sample 5 from the collection cup 2 into the aliquot container 30 and a mechanism of incorporating the aliquot container 30 into the collection cup 20. For these mechanisms, for example, a robot arm can be used.

If the liquid sample 5 is transferred into the aliquot container 30 and the aliquot container 30 is incorporated into the collection cup 20, the container 30 and cup 20 are conveyed as one unit to the aliquot/dispense unit 50 by the conveyance unit 40 as shown in FIG. 2.

The liquid sample 5 in the aliquot container 30 is aliquoted by the nozzle 52 of the aliquot/dispense unit 50. The aliquot container 30 is shaped like a cone and the housing space 35 is tapered.

Even though the amount of liquid sample 5 in the collection cup 20 is small, the sample 5 remains at the point portion of the housing space 35 if the sample 5 is transferred into the aliquot container 30. Therefore, the sample 5 can easily be aliquoted from the aliquot container 30 by the nozzle 52. The aliquoted sample 5 is dispensed into the inspection container.

In the sample preprocessing apparatus 10 so configured, the aliquot container 30 is used to aliquot a liquid sample. Since the housing space 35 in the aliquot container 30 is tapered, the liquid sample 5 remains at the point portion of the housing space 35. Hence, even though the amount of sample 5 is small, the sample 5 can be aliquoted with efficiency.

The inner surface 37 of the peripheral portion 34, by which the housing space 35 is formed in the aliquot container 30, extends linearly toward the point 36. If, therefore, the sample 5 adheres to the inner surface 37 of the peripheral portion 34 when the sample 5 is transferred from the collection cup 20 into the aliquot container 30, the adhering sample 5 moves to the point portion of the housing space 35 along the inner surface 37. Hence, even though the amount of sample 5 is small, the sample 5 remains at the point portion of the housing space 35, with the result that the sample 5 can be aliquoted with higher efficiency.

An aliquot container according to a second embodiment of the present invention will be described with reference to FIG. 5. In the second embodiment, the units having the similar functions as those of the units of the first embodiment are designated by the same reference numerals and their descriptions are not given. The main body section 31 of the second embodiment differs in shape from that of the first embodiment. The other configurations are the same as those of the first embodiment. Thus, only the difference will be described specifically.

Like FIG. 2, FIG. 5 is a cross-sectional view of a collection cup 20 and an aliquot container 30 incorporated into the collection cup 20. In FIG. 5, a sample is transferred from the collection cup 20 into the aliquot container 30.

As shown in FIG. 5, in the second embodiment, the main body section 31 of the aliquot container 30 is not shaped like a cone but tapered stepwise from an opening 33 of the main body section 31 to a point 36 thereof. Accordingly, the main body section 31 includes first, second, third and fourth step portions 71, 72, 73 and 74. The first step portion 71 communicates with the opening 31. The second step portion 72 communicates with the first step portion 71 and located closer to the point 36 than the first step portion 71. The area defined by the second step portion 72 is smaller than that defined by the first step portion 71.

The third step portion 73 communicates with the second step portion 72 and located closer to the point 36 than the second step portion 72. The area defined by the third step portion 73 is smaller than that defined by the second step portion 72, The fourth step portion 74 corresponds to a point portion of a housing space 35 in the main body section 31 and communicates with the third step portion 73. The area defined by the fourth step portion 74 is smaller than that defined by the third step portion 73. The first to fourth step portions 71 to 74 are shaped like a column. Each center of the first to fourth step portions 71 to 74 coincides with the center line C1.

The second embodiment brings about the same advantages as those of the first embodiment because the sample transferred into the aliquot container 30 remains in the fourth step portion 74 which corresponds to the point portion of the housing space 35 in the main body section 31.

In the first embodiment, the cross section of the housing space 35 that is an inner space of the main body section 31, taken along the line perpendicular to the center line C1 of the housing space 35, is a circle. In other words, the cross section of the inner space defined by the inner surface 37 of a peripheral portion 34 is a circle. The cross section gradually decreases in area toward the point 36. This is one example of the present invention in which the housing space gradually decreases toward the point from the opening. As another example, the cross section of the housing space, taken along the line perpendicular to the center line C1, can be shaped like a rectangle or it can be gradually decreased toward the point of the main body section, In other words, the cross section may have a shape other than a circle.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. An aliquot container comprising:
an opening (33); and
a main body section (31) which communicates with an outside through the opening (33) and which includes a peripheral portion (34) defining an inner space (35), the inner space (35) being tapered in a direction opposite to the opening (33).

2. The aliquot container according to claim 1, wherein the inner space (35) has a cross section taken along a line perpendicular to a center line of the main body section (31), and the Cross section gradually decreases toward a point of the main body section (31) from the opening (33).
